# EUROPEAN PATENT SPECIFICATION

(11) **EP 2 340 841 B1**
(45) Date of publication and mention of the grant of the patent: **09.11.2016**
(21) Application number: 09811231.1
(22) Date of filing: 05.08.2009
(51) Int. Cl.: A61K 31/663, A61K 9/08, A61K 47/02, A61P 1/02, A61P 19/08, A61P 43/00

(54) **OSTEOGENESIS PROMOTER COMPRISING [4-(METHYLTHIO)PHENYLTHIO]METHANEBISPHOSPHONIC ACID OR PHARMACEUTICALLY ACCEPTABLE SALT THEREOF AS ACTIVE INGREDIENT**
OSTEOGENESEPROMOTER MIT [4-(METHYLTHIO)PHENYLTHIO]METHANBISPHOSPHONSÄURE ODER PHARMAZEUTISCH AKZEPTABLEM SALZ DARAUS ALS WIRKSTOFF
PROMOTEUR D'OSTÉOGENÈSE COMPRENANT L'ACIDE [4-(MÉTHYLTHIO)PHÉNYLTHIO]MÉTHANEBISPHOSPHONIQUE OU SEL PHARMACEUTIQUEMENT ACCEPTABLE DE CELUI-CI EN TANT QU'INGRÉDIENT ACTIF

(30) Priority: 03.09.2008 JP 2008225484
(43) Date of publication of application: 06.07.2011
(73) Proprietor: Tohoku University, Aoba-ku Sendai-shi Miyagi 980-8577 (JP); Showa University, Tokyo, 1428555 (JP)
(72) Inventor: SHINODA, Hisashi, Sendai-shi Miyagi 980-8577 (JP); IGARASHI, Kaoru, Sendai-shi Miyagi 980-8577 (JP); MURAKAMI, shinobu, Sendai-shi Miyagi 980-8577 (JP); SUZUKI, Keiko, Tokyo 142-8555 (JP)
(74) Representative: Woods, Geoffrey Corlett
(86) International application number: PCT/JP2009/003758
(87) International publication number: WO 2010/026701

(56) References cited:
- EP-A1- 1 057 488
- WO-A1-00/38693
- WO-A1-01/08690
- WO-A1-01/17562
- WO-A1-93/05052
- WO-A1-94/19359
- US-A- 5 527 940
- IWASE M ET AL.: 'A novel bisphosphonate inhibits inflammatory bone resorption in a rat osteolysis model with continuous infusion of polyethylene particles' JOURNAL OF ORTHOPAEDIC RESEARCH vol. 20, 2002, pages 499 - 505, XP008147712
- KANGJUNG KIM: 'Osteolysis no Yakubutsugakuteki Yobo to Chiryo' JOURNAL OF JAPANESE SOCIETY FOR BIOMATERIALS vol. 23, no. 6, 2005, pages 390 - 395, XP008148008
- SHIBUTANI T ET AL: "Local application of bisphosphonate stimulates alveolar bone formation following tooth extraction", JOURNAL OF BONE AND MINERAL RESEARCH, vol. 15, no. Suppl. 1, September 2000 (2000-09), page S339, XP055091863, & TWENTY-SECOND ANNUAL MEETING OF THE AMERICAN SOCIETY FOR BONE AND MINERAL RESEARCH; TORONTO, ONTARIO, CANADA; SEPTEMBER 22-26, 2000 ISSN: 0884-0431
- BOBYN J D ET AL: "Zoledronic acid causes enhancement of bone growth into porous implants.", THE JOURNAL OF BONE AND JOINT SURGERY. BRITISH VOLUME MAR 2005, vol. 87, no. 3, March 2005 (2005-03), pages 416-420, XP009174868, ISSN: 0301-620X
- KAJIWARA H ET AL: "The bisphosphonate pamidronate on the surface of titanium stimulates bone formation around tibial implants in rats", BIOMATERIALS, ELSEVIER SCIENCE PUBLISHERS BV., BARKING, GB, vol. 26, no. 6, 1 February 2005 (2005-02-01), pages 581-587, XP027767834, ISSN: 0142-9612 [retrieved on 2005-02-01]
- TANZER MICHAEL ET AL: "The Otto Aufranc Award: bone augmentation around and within porous implants by local bisphosphonate elution.", CLINICAL ORTHOPAEDICS AND RELATED RESEARCH DEC 2005, vol. 441, December 2005 (2005-12), pages 30-39, XP009174866, ISSN: 0009-921X

## Description

### [Technical Field]

The present invention relates to an osteogenesis promoter or a pharmaceutical composition having [4-(methylthio)phenylthio] methanebisphosphonic acid, which is one type of bisphosphonic acid, or a pharmaceutically acceptable salt thereof as an active ingredient.

### [Background Art]

Bisphosphonic acid (BP, BPs) has a strong bone resorption inhibitory action resulting from inhibition of the function of osteoclasts, and to date, it has been widely used as a medical agent for various diseases with increased bone resorption such as osteoporosis, hypercalcemia, Paget disease, and neoplastic bone destruction.

Bisphosphonic acid is broadly categorized into, based on a chemical structure of side chains, (1) a compound group having an alkyl side chain, (2) a compound group having a halogen side chain, (3) a compound group having an aminoalkyl side chain, and (4) a compound group having a cyclic side chain. Various pharmacological properties of bisphosphonic acid such as its bone resorption inhibitory activity and the action mechanism thereof are known to vary significantly according to differences in the structure of these side chains.

For example, while a bisphosphonate compound (N-BPs: pamidronate, alendronate, risedronate, incadronate, zoledronate, etc.) having a nitrogen atom in the side chain inhibits a farnesyl pyrophosphate synthetase and a geranylgeranyl pyrophosphate synthetase in the mevalonate pathway and blocks prenylation of a low molecular G protein, a bisphosphonate compound (non N-BPs: etidronate, clodronate, tiludronate, etc.) not having the nitrogen atom in the side chain is known to have a high structural similarity with pyrophosphoric acid and form an ATP (adenosine triphosphate) analog. Moreover, N-BPs, compared to non N-BPs, is known to have a bone resorption inhibitory activity of 100-10,000 times or more.

Application of [4-(methylthio) phenylthio] methanebisphosphonic acid, which is one type of the bisphosphonate compounds not having a nitrogen atom in a side chain, to rheumatoid arthritis and an anti-inflammatory action are described in non-patent documents 1 to 4, and an improvement effect of bone metabolism disorders, an inhibitory effect of interleukin-1 production and action, and an antioxidative effect are described in the patent document 1 and non-patent document 5. Furthermore, it is known that this for periodontal disease (patent document 4 compound can be used as a medical agent and non-patent documents 6 and 7). However, the osteogenesis promoting action of the above compound was conventionally not known.

Moreover, the effect of bisphosphonic acid derivatives is variable, and it was well-known in the field of the technical field that when different bisphosphonic acids are used, the opposite effects may be caused and that even if the same bisphosphonic acid is used, according to the concentration, a varying biological reaction is caused (non-patent document 8).

On the other hand, as a substance having the osteogenesis promoting action, to date, BMP, FGF, IGF, and statins are well-known. However, for these substances, there was a problem in that because they are protein factors that promote osteogenesis and at the same time promote bone resorption (BMP) and that induce inflammation to application sites (BMP, statins), the manufacturing cost was high, antigenicity was a problem (BMP, FGF, IGF), affinity for a bone was low, special DDS (Drug Delivery System) was required (BMP, FGF, IGF, statins), etc.

### [Prior Arts]

### [Patent Document]

[Patent Document 1] Japanese Paten Publication Hei08-26048
[Patent Document 2] Japanese Patent No.3626500
[Patent Document 3] Japanese Patent No.2546067
[Patent Document 4] WO01/005403

### [Non-Patent Document]

[Non-Patent Document 1] Takaoka Y, Nagai H, Mori H, Tanahashi M: The effect of MPMBP on experimental arthritis in mice. Biol Pharmaco Bull 20:1147-50 (1997)
[Non-Patent Document 2] Tanahashi M, Funaba Y, Itoh M, Kawabe N, Nakadate-Matsushima T: Inhibitory effect of MPMBP on rat adjuvant arthritis. Pharmacology 56:242-251 (1998)
[Non-Patent Document 3] Tanahashi M, Koike J, Kawabe N, Nakadate-Matsushima T: Inhibitory effect of TRL-530 on inflammatory cytokines in bone marrow of rats with adjuvant arthritis. Pharmacology 56:237-241 (1998)
[Non-Patent Document 4] Iwase M, Kim KJ, Kobayashi Y, Itoh M, Itoh T: A novel bisphosphonate inhibits inflammatory bone resorption in a rat osteolysis model with continuous infusion of polyethylene particles. J Orthop Res 20:499-505 (2002)
[Non-Patent Document 5] Tanahashi M, Funaba Y, Tateishi A, Kawabe N, Nakadate-Matsushima T: MPMBP inhibits accumulation of superoxide anions derived from human polymorphonuclear leukocytes and bone resorption induced by activated osteoclasts. Pharmacology 56:125-130 (1998)
[Non-Patent Document 6] Sikder MN, Itoh M, Iwatsuki N, Shinoda H: Inhibitory effect of a novel bisphosphonate, MPMBP, on dental calculus formation in rats. J Periodontol 75:537-45 (2004)
[Non-Patent Document 7] Shinoda H, Takeyama S, Suzuki K, Murakami S, Yamada S: A novel bisphosphonate for the treatment of periodontitis. J Pharmacol Sci 106: 555-558 (2008)
[Non-Patent Document 8] Fleisch H: Experimental basis for the use of bisphosphonates in Paget's disease of bone. Clin Orthop Relat Res 217:72-78 (1987)

The following additional documents are acknowledged:
- US 5,527,940 which is concerned with a methanediphonate derivative, its manufacturing process and its pharmaceutical applications; and
- EP-A1-1,057,488 which is concerned with interleukin-6 production inhibitors.

### [Summary of the Invention]

### [Problems to be Solved by the Invention]

The present invention is intended to solve the problems described above. That is, the primary object of the present invention is to offer a compound that can be produced through a chemical synthesis at a low cost, that has an excellent osteogenesis promoting activity, that has high affinity for bones, and that can be applied without requiring any special DDS; and a method for promoting osteogenesis by administering the compound and for applying to the formation or regeneration of a bone.

### Means for Solving the Problems

The inventor of the present invention, carried out intensive studies in order to solve the above problems, and newly discovered that [4-(methylthio)phenylthio] methanebisphosphonic acid has an osteogenesis promoting action, which was not known to date, and completed the present invention.

That is, the present invention relates to aspects described below.
[Aspect 1] [4-(methylthio)phenylthio] methanebisphosphonic acid or a pharmaceutically acceptable salt thereof for use in a method of repairing, reconstructing and/or filling of: defective sites after removal of sockets, cysts, and tumors; alveolar bone defective sites absorbed by periodontal disease; alveolar clefts resulting from congenital anomaly; fractured sites; or peri-implant bones.
[Aspect 2] The salt for use of Aspect 1 which is a sodium salt.
[Aspect 3] The salt for use of Aspect 2 which is the disodium salt.
[Aspect 4] The acid for use of Aspect 1 or the salt for use of any one of Aspects 1 to 3 which is adsorbed to a sustained-releasing agent that is constituted from calcium phosphate.
[Aspect 5] The acid or salt for use according to Aspect 4, wherein calcium phosphate is selected from α-tricalcium phosphate, β-tricalcium phosphate, octa-calcium phosphate, and hydroxyapatite.
[Aspect 6] A pharmaceutical composition comprising [4-methylthio)phenylthio]methanebisphosphonic acid, or a salt thereof as defined in any one of Aspects 1 to 3, for use in a method of repairing, reconstructing and/or filling of: defective sites after removal of sockets, cysts, and tumors; alveolar bone defective sites absorbed by periodontal disease; alveolar clefts resulting from congenital anomaly; fractured sites; or peri-implant bones.
[Aspect 7] The pharmaceutical composition for use according to Aspect 6 in an aqueous formulation.
[Aspect 8] The pharmaceutical composition for use according to Aspect 7 in an injectable form.
[Aspect 9) The pharmaceutical composition for use according to any one of Aspects 6 to 8 wherein the said acid or salt is adsorbed to calcium phosphate as a sustained-releasing agent.
[Aspect 10] The pharmaceutical composition for use according to Aspect 9, wherein the calcium phosphate is selected from α-tricalcium phosphate, β-tricalcium phosphate, octa-calcium phosphate and hydroxyapatite.
[Aspect 11] The acid for use according to any one of Aspects 1, 4 or 5, the salt for use according to any one of Aspects 1 to 5, or the pharmaceutical composition for use according to any one of Aspects 6 to 10 wherein the said acid, salt or composition is administered locally to a region of a subject.
[Aspect 12] The acid, salt or pharmaceutical composition for use according to Aspect 10 or 11, wherein the said region is alveolar bone site.

### [Advantage of the Invention]

By causing [4-(methylthio)phenylthio] methanebisphosphonic acid, which is one type of bisphosphonic acid, or the pharmaceutically acceptable salt thereof to act, osteogenesis promotion was observed in a surrounding site of administration in the living body, in osteoblast-like cell lines, expression of alkaline phosphatase (ALP) activity and/or osteogenesis related genes was increased significantly, and it was possible to induce, in the bone organ culture system, promotion of collagen synthesis, promotion of osteogenesis, and/or promotion of production of the bone matrix, and increase of the bone mass, significantly.

### [Brief Description of the Drawings]

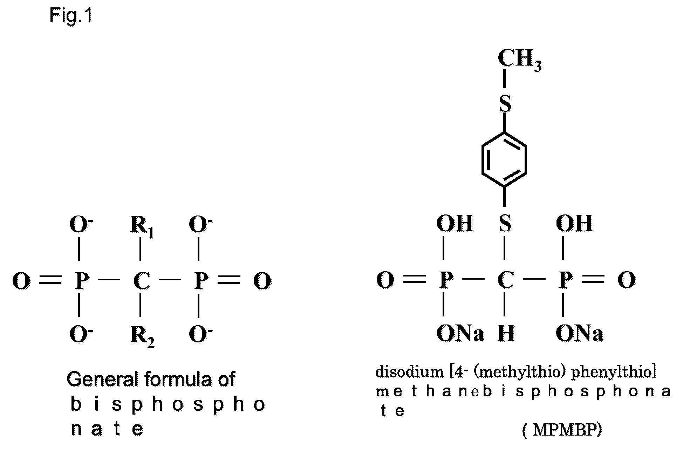

[Fig. 1] is a general expression of bisphosphonate and a chemical structure of [4-(methylthio)phenylthio]-methanebisphosphonic acid • disodium salt (abbreviation: "MPMBP".

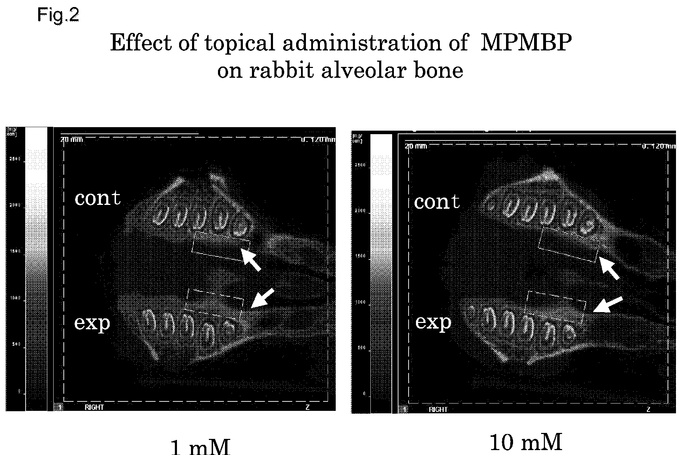

[Fig. 2] is a proliferation of the bone induced on the maxillary alveolar bone of a rabbit resulting from local administration of MPMBP (administration of a 1 mM or 10 mM solution every 4 days for 6 times). (pQCT: peripheral Quantitative Computerized Tomography image). A horizontal section in the vicinity of the cervical site of the maxillary molar teeth. Exp: A test side (side to which MPMBP was administered), cont: a control side (side to which 0.9% NaCl was administered). Arrows represent injected sites. On the test side, in the middle side of the palate, a proliferation of the alveolar bone is observed.

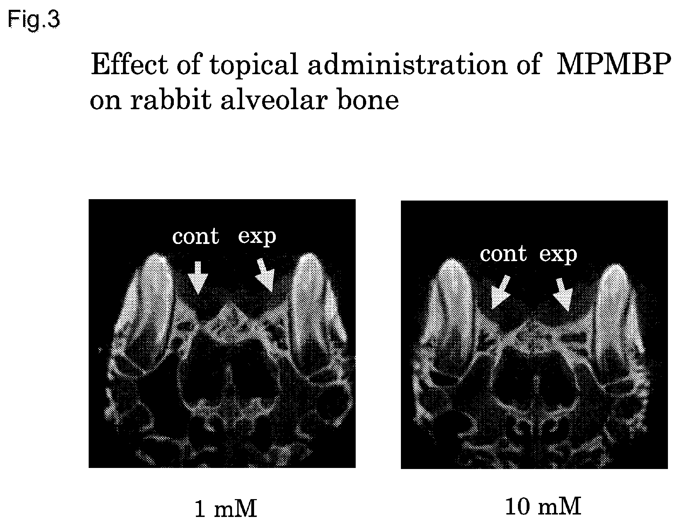

[Fig. 3] is a proliferation of the bone induced on the maxillary alveolar bone of a rabbit resulting from local administration of MPMBP (administration of a 1 mM or 10 mM solution every 4 days for 6 times) (µCT image: microfocus Computerized Tomography image). A forehead cut of the midline region of the right and left maxillary first molars. exp: A test side (side to which MPMBP was administered). cont: A control side (side to which 0.9% NaCl was administered). From the alveolar bone of the test side to the middle side of the palate, the thickness of the palatine bone is increasing.

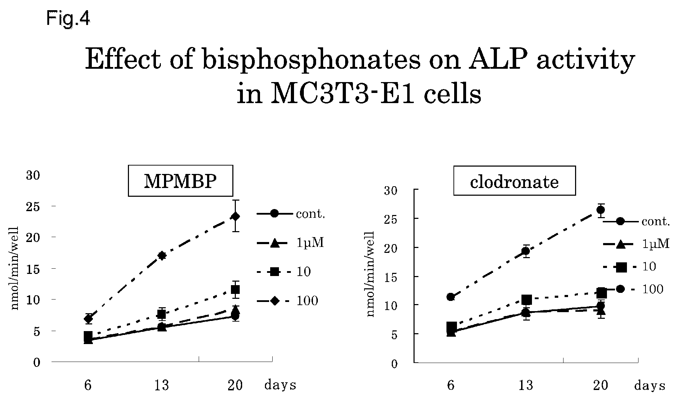

[Fig. 4] is an effect of MPMBP (1, 10, and 100 µM) on the osteoblast lineage cell lines MC3T3-E1 cells. MPMBP and clodronate (one of non-nitrogen-containing bisphosphonate), during the culture period of 3 weeks, increase an alkaline phosphatase activity in a dose-dependent manner and with time.

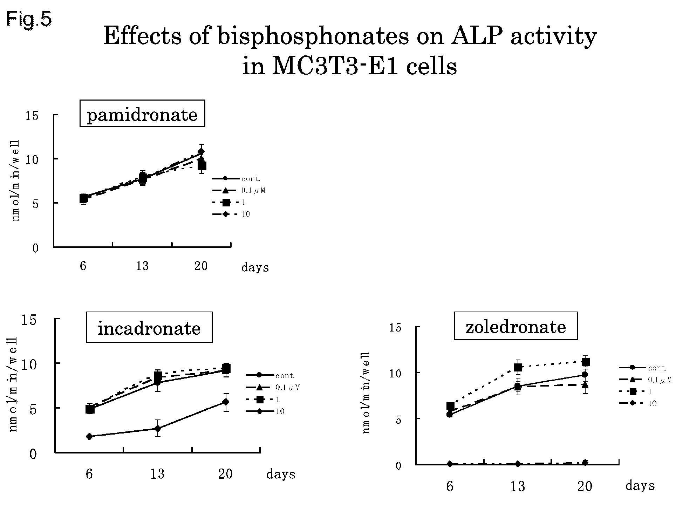

[Fig. 5] is an effect of bisphosphonate (non-nitrogen-containing bisphosphonate, namely pamidronate, incadronate, and zoledronate. Respectively, 0.1, 1, and 10 µM) on the osteoblast-like cell line MC3T3-E1 cells. Unlike MPMBP, the alkaline phosphatase activity is inhibited or the alkaline phosphatase activity is not affected.

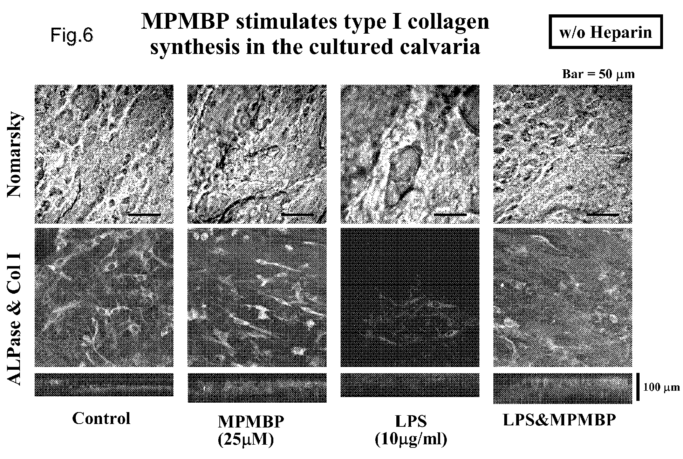

[Fig. 6] is a promoting activity of production of the bone matrix of MPMBP in the bone organ culture system of a mouse. After a type I collagen of the cultured bone is stained immunohistochemically, it was observed using a confocal laser scanning microscope. The upper panels are Nomarski differential interference contrast images of the bone surface. The middle panels are images of the bone surface after a double staining of the type I collagen and alkaline phosphatase was performed. The lower panels are images of the bone section after the double staining of the type I collagen and alkaline phosphatase was performed. Red color (rhodamine): type I collagen. Green color (ELF-97): alkaline phosphatase (osteoblast). Results after cultured for 48 hours under the presence of MPMBP using DMEM. Regardless of the presence or absence of LPS (lipopolysaccharide: one of the bone resorption promoting factors), MPMBP, compared to the control bone, production of the bone matrix (sites in red color) was promoted significantly. Moreover, based on the observation of the Nomarski differential interference contrast images, under the presence of MPMBP, the bone resorption was inhibited resulting from LPS and the size of a resorption cavity that was formed from LPS was significantly smaller, compared to the control bone.

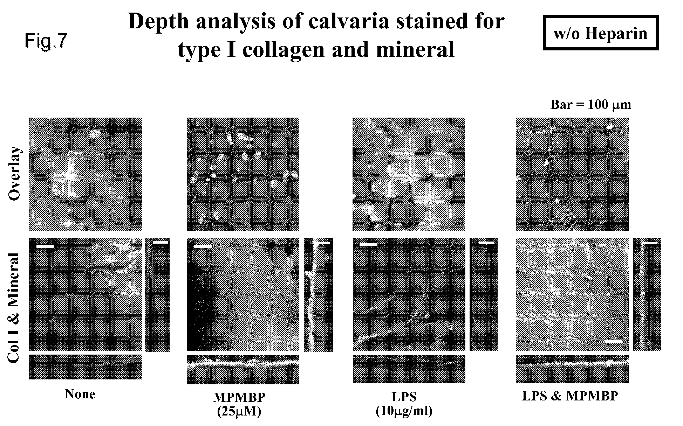

[Fig. 7] is a promoting activity of production of the bone matrix and an increasing effect of the bone mass of MPMBP in the bone organ culture system of a mouse. After the type I collagen of the cultured bone is stained immunohistochemically, it was observed using a confocal laser scanning microscope. The upper panels are Nomarski differential interference contrast images of the bone surface. The middle panels are images of the bone surface after a double staining of type I collagen and alkaline phosphatase was performed. The lower panels are images of the bone section after the double staining of the type I collagen and alkaline phosphatase was performed. Red color (alizarin red): bone mineral. Green color (Alexa Fluor-488): type I collagen. Results after cultured for 48 hours under the presence of MPMBP using DMEM. Regardless of the presence or absence of LPS (lipopolysaccharide: one of the bone resorption promoting factors), MPMBP, compared to the control bone, production of the bone matrix (sites in green color) was promoted significantly. Moreover, the thickness of the bone mineral (sites in red color) was thick, compared to the control bone. Furthermore, based on the observation of the Nomarski differential interference contrast images, under the presence of MPMBP, the bone resorption was inhibited resulting from LPS and the size of the resorption cavity that was formed from LPS was significantly smaller, compared to the control bone.

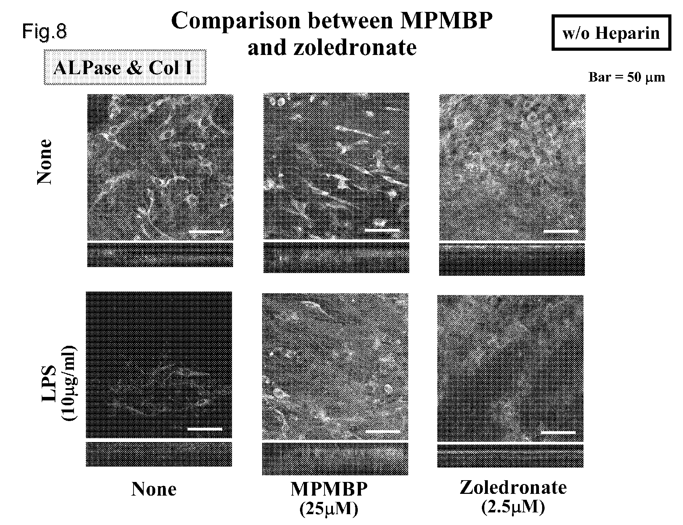

[Fig. 8] is a promoting activity of production of the bone matrix of MPMBP in the bone organ culture system of a mouse. Comparison with zoledronate. After a double staining was performed on the type I collagen and alkaline phosphatase of the cultured bone, it was observed with a confocal laser scanning microscope. Respectively, the upper panels are observation images of the bone surface and the lower panels are images of the bone section. Results after cultured for 48 hours under the presence of MPMBP using DMEM. Red color (rhodamine): type I collagen. Green color (ELF-97): alkaline phosphatase (osteoblasts). In both the presence and absence of LPS, zoledronate inhibited production of the type I collagen, and in contrast, MPMBP significantly promoted production of the bone matrix. In the MPMBP supplementation group, resulting from a large amount of collagen fibers that are secreted by osteoblasts, osteoblasts themselves were covered, making it difficult to observe, and in contrast in the zoledronate supplementation group, because production of collagen was inhibited, osteoblasts (green color) could be observed as is.

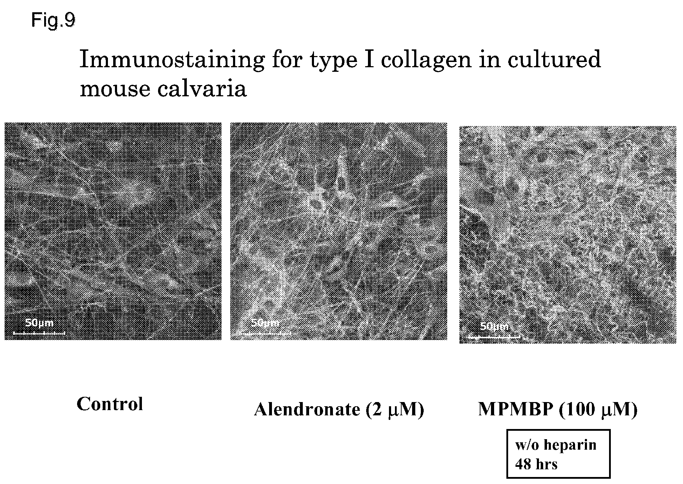

[Fig. 9] is a promoting activity of production of the bone matrix of MPMBP in the bone organ culture system of a mouse. Comparison with alendronate. After the type I collagen of the cultured bone was stained immunohistochemically, it was observed with a confocal laser scanning microscope. The collagen fibers and osteoblasts were stained in green color by Alexa Fluor-488. Comparison with alendronate, which is the only one in which the osteogenesis promoting action was reported among the bisphosphonate compound. Cultured for 48 hours using DMEM. For alendronate, as was the case with MPMBP, compared to the control bone, production of collagen was increased; however, the degree was small, compared to MPMBP. Under the presence of MPMBP, a large amount of thick collagen fibers were produced in a fascicular shape and the bone surface was densely covered, and in contrast, the collagen fibers that are produced under the presence of alendronate was thin and had a needle shape, and the amount was less, compared to MPMBP.

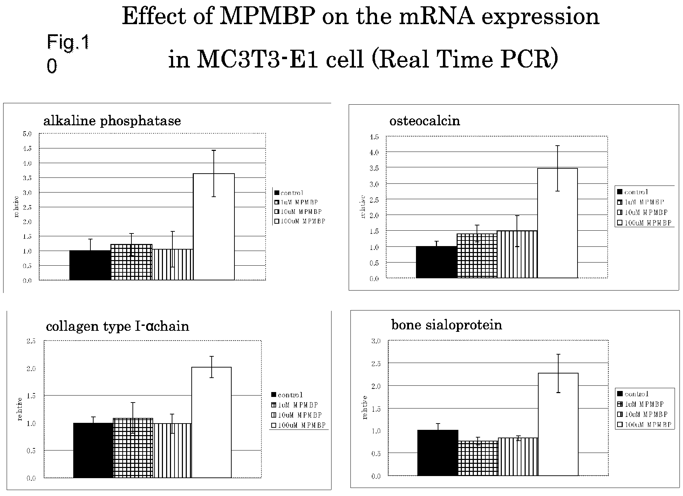

[Fig. 10] is an effect of MPMBP on expression of osteogenesis-related genes. After the osteoblast-like cell line MC3T3-E1 cells were cultured under the presence of MPMBP (1, 10, and 100 µM) for 72 hours, mRNA was extracted. Gene expression of alkaline phosphatase, type 1-α collagen, osteocalcin, and bone sialoprotein was analyzed using the real-time RT-PCR method. MPMBP also promoted gene expression of all of the above. The respective bars in the figures show, from the left to the right, respectively, the results of the control, 1, 10, and 100 µM.

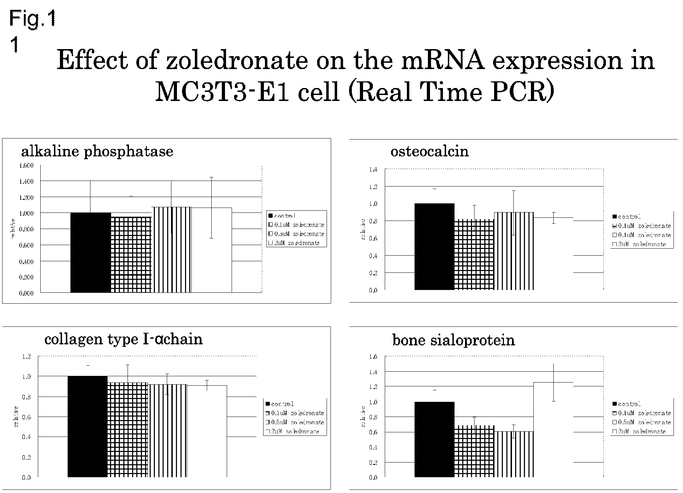

[Fig. 11] is an effect of zoledronate on expression of osteogenesis-related genes. After the osteoblast-based cell line MC3T3-E1 cells were cultured under the presence of MPMBP (1, 10, and 100 µM) for 72 hours, mRNA was extracted. Gene expression of alkaline phosphatase, type 1-α collagen, osteocalcin, and bone sialoprotein was analyzed using the real-time RT-PCR method. Zoledronate, unlike MPMBP, did not significantly affect gene expression of all of the above. The respective bars in the figures show, from the left to the right, respectively, the results of the control, 1, 10, and 100 µM.

### [Mode for Carrying out the Invention]

The present invention relates to [4-(methylthio)phenylthio] methanebisphosphonic acid, a pharmaceutically acceptable salt thereof or a pharmaceutical composition comprising having [4-(methylthio)phenylthio] methanebisphosphonic acid, which is one type of bisphosphonic acid, or a pharmaceutically acceptable salt thereof as an active ingredient.

In the present invention, an "osteogenesis promoting action" refers to at least one of an alkaline phosphatase (ALP) activity that is known as the index for differentiating osteoblast precursor cells to osteoblasts in the osteogenesis promotion in the living body and in the culture system of the osteoblast-like cell lines; and/or an activity to significantly increase the expression of osteogenesis-related genes of type-I collagen, osteocalcin, bone sialoprotein, etc., known as other index for promoting osteogenesis; an activity, in the bone organ culture system such as the skull cap, that induces promotion of collagen synthesis, promotion of osteogenesis, and/or promotion of production of the bone matrix, and increases of the bone mass; and an activity that may be considered to be practically the same or is correlated physiologically or pharmacologically to these activities.

[4-(methylthio)phenylthio] methanebisphosphonic acid or a pharmaceutically acceptable salt thereof, for example, can be manufactured with any known method to those skilled in the art, as described in the patent documents 1 to 3.

As a salt that may be a pharmaceutically acceptable for [4-(methylthio)phenylthio] methanebisphosphonic acid, any known salt to those skilled in the art, for example, sodium salt, potassium salt, etc., can be mentioned. As a specific example, [4-(methylthio)phenylthio] methanebisphosphonic acid (abbreviation: "MPMBP") showing the structural formula in Fig. 1 can be mentioned.

[4-(methylthio)phenylthio] methanebisphosphonic acid or pharmaceutically acceptable salt thereof is useful as a pharmaceutical composition having various drug formulations such as forms of oral administration such as a tablet, a capsule, a powdered drug, a granule, and a pill, and such as forms of non-oral administration such as an injection, a syrup, an ointment, a buccal tablet, a suppository, an oral cleaning agent, and a topical drug, and the pharmaceutical composition shows osteogenesis promoting action.

To the pharmaceutical composition of the present invention, by taking into consideration of the above drug formulations, aside from the active ingredients of the present invention, a carrier, an excipient, a bonding agent, a lubricant, a disintegrator, a sustained-releasing agent, a buffer, a coating agent, a coloring agent, etc., which may be pharmaceutically acceptable to those skilled in the art, can be included as appropriate. The [4-(methylthio)phenylthio] methanebisphosphonic acid or pharmaceutically acceptable salt thereof or the pharmaceutical composition of the present invention can be formulated easily with any known manufacturing method to those skilled in the art.

For example, examples of appropriate carriers include lactose, starch, sucrose, glucose, methylcellulose, magnesium stearate, mannitol, sorbitol, and croscarmellose sodium. Or, appropriate bonding agents include starch, gelatin, or glucose, anhydrous lactose, free-flow lactose, β-lactose, natural sugar such as a corn sweetener, gum Arabic, guar gum, a natural or synthetic gum such as tragacanth or sodium alginate, carboxymethyl-cellulose, polyethylene glycol, wax, etc. Moreover, lubricants used as the dosage for of these include sodium oleate, sodium stearate, magnesium stearate, sodium benzoate, sodium chloride, etc. Furthermore, in order to orally administer in the liquid form such as an elixir, syrup, and a form composition, oral drug components can be combined with pharmaceutically acceptable non-toxic oral non-active carriers, such as ethanol, glycerol, and water. Moreover, to the pharmaceutical carriers that are constituted from soluble polymers such as polyvinylpyrrolidone, pyran polymer, polyhydroxypropylmethacrylamide, the active ingredients may be combined.

As a preferred example of the pharmaceutical composition of the present invention, a drug formulation that, in the state in which the active ingredients are adsorbed to the sustained-releasing agent that is constituted from calcium phosphate such as α-tricalcium phosphate, β-tricalcium phosphate, octa-calcium phosphate, and hydroxyapatite, is included in an aqueous solution in which the osmotic pressure and pH are adjusted to the physiological range, can be mentioned. Moreover, it is preferable that the osmotic pressure and pH of this aqueous solution are adjusted to the physiological range.

The content and the dose of the active ingredients that are contained in the pharmaceutical composition of the present invention can be selected by those skilled in the art appropriately, according to an administration plan, an oral drug of specified bisphosphonic acid compound that is selected, the age of recipient patients,
size, gender and physical condition, type and the degree of seriousness of disorders to be treated, and other relevant medical and physical factors. Moreover, the appropriate amount can be determined based on a typical experiment of an animal model and on a clinical test on human. Generally, the appropriate amount of the active ingredients is selected such that significant osteogenesis promoting effects are obtained.

Therefore, although the dose, administration intervals, etc., can be determined appropriately according to an administration subject, administration route, symptoms, etc., with respect to the amount of the active ingredients, it is approximately 0.1 mg to 5 g, preferably approximately 1 mg to 2 g, and by dividing it into one to several times daily, appropriate duration, for example, for 1 day to 30 days, oral or non-oral administration is performed. For example, an injection can be locally administered to treatment sites. Moreover, although the type, sites, etc., of bones to which formation is promoted according to the present invention are not limited, as the treatment sites or the surrounding sites, for example, an alveolar bone, a peri-implant bone, a site after removal of bone cyst, etc., can be mentioned.

Although the present invention is described in detail below with reference to Examples, these Examples are simply for explanation purposes only, and they are intended to provide as references of the specific Examples. Although these examples are intended to provide explanations of specific detailed Examples of the present invention, they are not intended to limit or restrict the scope of the invention disclosed in the present specification. In the present invention, it should be understood that various Examples are possible based on the idea of the present invention. Moreover, for cases in which a commercially available reagent or kit is used, the protocols attached to those, drugs attached, etc., are used.

### [Examples]

### Example (1)

A total of 9 Japanese male rabbits with the average weight of 2.2 kg were used and they were divided into 3 groups of 3 rabbits each. Under the periosteum of the palatal alveolar bone between the teeth of the maxillary first molar and the maxillary second molar on the left side (experiment side) of the domestic rabbits in each group, 50 µl of a 0.1, 1.0, or 10 mM MPMBP solution was injected every 4 days for 6 times (all performed under anesthesia using Nembutal). To the same sites on the opposite side (the right side), 50µl of a physiological saline solution (0.9% NaCl solution) was injected in the same manner, and was set to be a control. On the 4th day after the final injection, the animals were euthanized with excessive administration of Nembutal, and the maxillary bone was removed and fixed with a 10% neutral formalin solution (pH 7.4). With the alveolar bone in the vicinity of drug administration sites and the palatine bone as the region of interest, 3-dimensional analysis was performed using a pQCT (peripheral Quantitative Computerized Tomography; Fig. 2) and a µCT(microfocus Computerized Tomography; Fig. 3). The results were such that, in the domestic rabbits in the group in which 10 mM and 1.0 mM of MPMBP were administered, images in which the bone addition to the palatal side of the alveolar bones (parts in the arrows shown in "exp") as shown in Fig. 2 were observed and images in which the thickness of the palatine bone increased from the alveolar site to the middle site of the palate were observed (parts in the arrows shown in "exp"). For these changes, compared to the 1.0 mM administration group, it was stronger for the 10 mM administration group, and the effect was not clear in the 0.1 mM administration group. This type of the osteogenesis promoting effect of MPMBP in the living body has not been reported with the existing bisphosphonate compound.

### Example (2)

Using the mouse skull-derived osteoblast-like cell line MC3T3-E1 cells (RIKEN Bio Resource Center, CELL BANK: RCB1126), with respect to the alkaline phosphatase (ALP) activity, known as one index of osteogenesis, and also known as the index of differentiation of osteoblast precursor cells to osteoblasts, what kind of effects MPMBP has was studied. After MC3T3-E1 cells were cultured in a α-MEM (10% bovine serum supplementation) and after the confluent was reached, under the presence or absence of MPMBP (1, 10, and 100 µM), for 6, 10, or 20 days, they were cultured in the culture plate of 48 wells. After completion of culturing, a cell homogenate was prepared, and using the method by Lowry et al (J Biochem 1954; 207: 19-37), the ALP activity (per well) was measured. The results were such that MPMBP increased the ALP activity in a dose-dependent manner and with time (Fig. 4, left). When the same experiment was performed with regard to other bisphosphonate compound (clodronate, pamidronate, incadronate, zoledronate), the results were such that clodronate, as is known, (Felix and Fleisch, Biochem J 1979; 183:73-81; Igarashi et al, Prostaglandins, Leukotriens, and Essential Fatty Acids 1997; 56: 12-125) increased the ALP activity (Fig. 4, right); however, no significant change was observed with pamidronate (Fig. 5, upper left), and with incadronate and zoledronate, the ALP activity was significantly reduced in high concentration (Fig. 5, lower left and lower right). These facts suggest that the effect of the bisphosphonate compound on the ALP activity is not the same and depending on what kind of side chains are added to a P-C-P combined carbon atom, it differs, and at the same time, these facts suggest that in MPMBP, [4-(methylthio)phenylthio] side chains play an important role in increasing the ALP activity.

### Example (3)

Using the bone organ culture system, what kind of effects MPMBP has on production of the bone matrix was studied. From 3-6 day old mice, calvariae were collected aseptically. These were cultured using the Stern and Krieger method (Calcif Tissue Int 1983; 35: 172-17) with DMEM (10% bovine serum supplementation, no supplementation of heparin, supplementation or no supplementation of LPS 10 µg/ml) for 48 hours. After completion of culturing, with the method described by Suzuki, Takeyama et al (J Histochem Cytochem 2005; 53: 1525-1537), a double staining of the type I collagen and alkaline phosphatase (Fig. 6 and Fig. 8), a double staining of the type I collagen and alizarin red (Fig. 7), and an immunohistochemical staining using the type I collagen (Fig. 9) were performed, and using a confocal laser scanning microscope, they were observed.

Fig. 6 shows the promoting activity of production of the bone matrix of MPMBP (25 µM) in the bone organ culture system of a mouse. The upper panels are Nomarski differential interference contrast images of the bone surface. The middle panels are images of the bone surface after the double staining of the type I collagen and alkaline phosphatase was performed, and the lower panels are images of the bone section after the double staining of the type I collagen and alkaline phosphatase was performed, respectively. Using rhodamine for the type I collagen, red color could be observed, and using a substrate (ELF-97) that fluoresces when dephosphorylated for alkaline phosphatase (stain osteoblasts), the enzyme activity could be observed in green color. By adding MPMBP into a culture fluid, regardless of the presence or absence of LPS (10 µg/ml) (lipopolysaccharide: one of the bone resorption promoting factors), compared to the control bone, production of the bone matrix (type I collagen: sites in red) significantly increased. For osteoblasts, under the presence of MPMBP, production of the collagen fibers increased; therefore, they were covered with the collagen fibers that they themselves produced, and no clear observation could be made, as was the case with the control bone. Moreover, based on the observation of the Nomarski differential interference contrast images, under the presence of MPMBP, it can be observed that the bone resorption was inhibited resulting from LPS and the size of the resorption cavity that was formed by LPS was significantly small, compared to the control bone.

Fig. 7 shows the promoting activity of production of the bone matrix and the increasing effect of the bone mass of MPMBP in the bone organ culture system of a mouse. The upper panels are Nomarski differential interference contrast images of the bone surface. The middle panels are images of the bone surface after the type I collagen staining was performed, and in this figure, the collagen fibers were observed in green color resulting from Alexa Fluor-488. The lower panels are images of the bone section after the double staining of the type I collagen and alizarin red was performed, and the collagen fibers could be observed in green color resulting from Alexa Fluor-488 and the bone mineral could be observed in red color resulting from alizarin red. When cultured under the presence of MPMBP for 48 hours, the results were such that regardless of the presence or absence of LPS, compared to the control bone, images in which, compared to the control bone, production of the bone matrix (sites in green color) significantly increased, were observed. Moreover, the thickness of the bone mineral (sites in red color) was thick, compared to the control bone. Furthermore, based on the observation of the Nomarski differential interference contrast images, under the presence of MPMBP, the bone resorption was inhibited resulting from LPS and the size of the resorption cavity that was formed by LPS was significantly small, compared to the control bone.

Fig. 8 shows the results of comparison of effects of MPMBP and zoledronate (2.5 µM) in the same bone organ culture system. After the immunological double staining of the type I collagen and alkaline phosphatase was performed, as was the case with Fig. 6, using Alexa Fluor 594 for the type I collagen, it was stained in red color, and using the substrate (ELF-97) that fluoresces when dephosphorylated for alkaline phosphatase (osteoblasts), the enzyme activity was stained in green color. The upper panels are observation images of the bone surface and the lower panels are images of the bone section. Zoledronate, regardless of the presence or absence of LPS, inhibited production of the type I collagen, and in contrast, MPMBP significantly increased production of the bone matrix. In the MPMBP supplementation group, resulting from a large amount of the collagen fibers secreted from the osteoblasts, osteoblasts themselves were covered, making it difficult to observe, and in contrast, in the zoledronate supplementation group, because production of the collagen was inhibited, osteoblasts (green color) could be easily observed.

Fig. 9 shows the results in which the promoting activity of production of the bone matrix of MPMBP was compared with the effect of alendronate, which is the only one in which the osteogenesis promoting action is reported among the bisphosphonate compound (Patent Number 3566984 osteogenesis promoter; Tsuchimoto, Azuma et al, Jpn J Pharmacol 1994; 66: 25-33; Duque and Rivas, J Bone Miner Res 2007; 22: 1603-1611). It shows the results in which after culturing using the concentration indicating the same level of the inhibitory action of the bone resorption in the bone organ culture system (MPMBP: 100 µM; alendronate : 2.0 µM) for 48 hours (no supplementation of heparin), the type I collagen was immunologically stained and they were stained in green color by Alexa Fluor-488. Even under the presence of alendronate, as was the case with MPMBP, compared to the control bone, images in which production of the collagen increased were observed; however, the degree was small, compared to MPMBP. Under the presence of MPMBP, a large amount of thick collagen fibers was produced in a fascicular shape and the bone surface was densely covered, and in contrast, for the collagen fibers that were produced under the presence of alendronate, there were many that were thin and had a needle shape. With alendronate, because the amount of the collagen fibers produced is not as large as those with MPMBP, osteoblasts could be easily observed.

As above, zoledronate works such that production of the bone matrix is inhibited, and in contrast, it was clear that MPMBP acts such that it increases production of the bone matrix. Moreover, the degree is significantly higher, compared to the effect of alendronate, which is the only one in which the osteogenesis promoting action is reported.

### Example (4)

As an experiment to clarify the mechanism of the osteogenesis promoting action of MPMBP, using the osteoblast-based cell line MC3T3-E1 cells, the effect on expression of osteogenesis-related genes was studied using the real-time RT-PCR method. Moreover, the effect was compared with the action of zoledronate.

In the experiment, the osteoblast-based cell line MC3T3-E1 cells that reached the confluent after cultured with α-MEM (10% bovine serum supplementation) were used. For 3 days, they were cultured (used a dish with the diameter of 60 mm) under the presence of MPMBP(1, 10, or 100µM) or zoledronate (0.1, 0.5 or 2.0 µM). After completion of culturing, cells were scraped and using Trizol, RNA was extracted. From 5 µg of RNA, cDNA was synthesized, specific primers were set with respect to alkaline phosphatase, the type I-α collagen, osteocalcin, and bone sialoprotein genes, PCR amplification was performed in the thermal cycler, and the amount of expression of the above genes was measured.

The results were such that for MPMBP, at the concentration of 100 µM, expression of the above genes known to be the index for promotion of osteogenesis was increased significantly in all genes (Fig. 10). In contrast, for zoledronate, within the concentration range (concentration range in which the cell proliferation is not inhibited), no significant change was caused to the amount of expression of these genes (Fig. 11).

As above, for all four Examples, it was newly confirmed that MPMBP has the promoting effects with respect to osteogenesis.

### [Industrial Applicability]

[4-(methylthio)phenylthio] methanebisphosphonic acid or the pharmaceutically acceptable salt thereof is provided for use in a method of repairing, reconstructing and/or filling of: defective sites after removal of sockets, cysts, and tumors; alveolar bone defective sites absorbed by periodontal disease; alveolar clefts resulting from congenital anomaly; fractured sites; or peri-implant bones

## Claims

1. [4-(Methylthio)phenylthio]methanebisphosphonic acid or a pharmaceutically acceptable salt thereof for use in a method of repairing, reconstructing and/or filling of: defective sites after removal of sockets, cysts, and tumors; alveolar bone defective sites absorbed by periodontal disease; alveolar clefts resulting from congenital anomaly; fractured sites; or peri-implant bones.

2. The salt for use according to claim 1 which is a sodium salt.

3. The salt for use according to claim 2 which is the disodium salt.

4. The acid for use according to claim 1 or the salt for use according to any one of the preceding claims which is adsorbed to calcium phosphate as a sustained-releasing agent.

5. The acid or salt for use according to claim 4 wherein the calcium phosphate is selected from α-tricalcium phosphate, β-tricalcium phosphate, octa-calcium phosphate and hydroxyapatite.

6. A pharmaceutical composition comprising [4-(methylthio)phenylthio]methanebisphosphonic acid, or a salt thereof as defined in any one of claims 1 to 3, for use in a method of repairing, reconstructing and/or filling of: defective sites after removal of sockets, cysts, and tumors; alveolar bone defective sites absorbed by periodontal disease; alveolar clefts resulting from congenital anomaly; fractured sites; or peri-implant bones.

7. The pharmaceutical composition for use according to claim 6 in an aqueous formulation.

8. The pharmaceutical composition for use according to claim 7 in an injectable form.

9. The pharmaceutical composition for use according to any one of claims 6 to 8 wherein the said acid or salt is adsorbed to calcium phosphate as a sustained-releasing agent.

10. The pharmaceutical composition for use according to claim 9, wherein the calcium phosphate is selected from α-tricalcium phosphate, β-tricalcium phosphate, octa-calcium phosphate and hydroxyapatite.

11. The acid for use according to any one of claims 1, 4 or 5, the salt for use according to any one of claims 1 to 5, or the pharmaceutical composition for use according to any one of claims 6 to 10, wherein the said acid, salt or composition is administered locally to a region of a subject.

12. The acid, salt or pharmaceutical composition for use according to claim 10 or 11 , wherein the said region is an alveolar bone region.

## Patentansprüche

1. [4-(Methylthio)phenylthio]methanbisphosphonsäure oder ein pharmazeutisch verträgliches Salz davon zur Verwendung bei einem Verfahren zur Reparatur, Rekonstruktion und/oder Füllung von:
fehlerhaften Stellen nach Entfernen von Gelenkpfannen, Zysten, Tumoren; alveolären Knochen-Mangelstellen, die von Paradontalkrankheit absorbiert werden; alveolären Rissen auf Grund kongenitaler Anomalie;
Frakturstellen; oder Periimplantatknochen.

2. Salz zur Verwendung nach Anspruch 1, das ein Natriumsalz ist.

3. Salz zur Verwendung nach Anspruch 2, das das Dinatriumsalz ist.

4. Säure zur Verwendung nach Anspruch 1 oder das Salz zur Verwendung nach einem der vorangehenden Ansprüche, das an Calciumphosphat adsorbiert ist, als Mittel mit verzögerter Freisetzung.

5. Säure oder Salz zur Verwendung nach Anspruch 4, wobei das Calciumphosphat ausgewählt ist aus α-Tricalciumphosphat, β-Tricalciumphosphat, Octacalciumphosphat und Hydroxylapatit.

6. Pharmazeutische Zusammensetzung, umfassend [4-(Methylthio)phenylthio] methanbisphosphonsäure oder ein Salz davon nach einem der Ansprüche 1 bis 3, zur Verwendung bei einem Verfahren zur Reparatur, Rekonstruktion und/oder Füllung von: fehlerhaften Stellen nach Entfernen von Gelenkpfannen, Zysten, Tumoren; alveolären Knochen-Mangelstellen, die von Paradotalkrankheit absorbiert werden; alveolären Rissen auf Grund kongenitaler Anomalie;
Frakturstellen; oder Periimplantatknochen.

7. Pharmazeutische Zusammensetzung zur Verwendung nach Anspruch 6 in einer wässrigen Formulierung.

8. Pharmazeutische Zusammensetzung zur Verwendung nach Anspruch 7 in einer injizierbaren Form.

9. Pharmazeutische Zusammensetzung zur Verwendung nach einem der Ansprüche 6 bis 8, wobei die Säure oder das Salz an Calciumphosphat adsorbiert ist, als Mittel mit verzögerter Freisetzung.

10. Pharmazeutische Zusammensetzung zur Verwendung nach Anspruch 9, wobei das Calciumphosphat aus α-Tricalciumphosphat, β-Tricalciumphosphat, Octacalciumphosphat und Hydroxylapatit ausgewählt ist.

11. Säure zur Verwendung nach einem der Ansprüche 1, 4 oder 5, das Salz zur Verwendung nach einem der Ansprüche 1 bis 5, oder die pharmazeutische Zusammensetzung zur Verwendung nach einem der Ansprüche 6 bis 10, wobei die Säure, das Salz oder die Zusammensetzung lokal an eine Region eines Testindividuums verabreicht wird.

12. Säure, Salz oder die pharmazeutische Zusammensetzung zur Verwendung nach Anspruch 10 oder 11, wobei die Region eine alveoläre Knochenregion ist.

## Revendications

1. Acide [4-(méthylthio)phénylthio]méthanebisphosphonique ou sel pharmaceutiquement acceptable de celui-ci, pour utilisation dans un procédé de réparation, reconstruction et/ou comblement de : sites défectifs après extraction d'emboîtures, de kystes et de tumeurs ; sites défectifs d'os alvéolaires engendrés par parondotolyse ; fentes alvéolaires résultant d'une anomalie congénitale ; sites fracturés ; ou os péri-implantaires.

2. Sel pour utilisation selon la revendication 1, qui est un sel de sodium.

3. Sel pour utilisation selon la revendication 2, qui est le sel disodique.

4. Acide pour utilisation selon la revendication 1 ou sel pour utilisation selon l'une quelconque des revendications précédentes, qui est adsorbé sur du phosphate de calcium en tant qu'agent de libération prolongée.

5. Sel ou acide selon la revendication 4, le phosphate de calcium étant choisi parmi le phosphate tricalcique α, le phosphate tricalcique β, le phosphate octocalcique et l'hydroxyapatite.

6. Composition pharmaceutique comprenant de l'acide [4-(méthylthio)-phénylthio]méthanebisphosphonique ou un sel de celui-ci, tel que défini dans l'une quelconque des revendications 1 à 3, pour utilisation dans un procédé de réparation, reconstruction et/ou comblement de : sites défectifs après extraction d'emboîtures, de kystes et de tumeurs ; sites défectifs d'os alvéolaires engendrés par parondotolyse ; fentes alvéolaires résultant d'une anomalie congénitale ; sites fracturés ; ou os péri-implantaires.

7. Composition pharmaceutique pour utilisation selon la revendication 6, dans une formulation aqueuse.

8. Composition pharmaceutique pour utilisation selon la revendication 7, sous une forme injectable.

9. Composition pharmaceutique pour utilisation selon l'une quelconque des revendications 6 à 8, dans laquelle ledit acide ou sel est adsorbé sur du phosphate de calcium en tant qu'agent de libération prolongée.

10. Composition pharmaceutique pour utilisation selon la revendication 9, dans laquelle le phosphate de calcium est choisi parmi le phosphate tricalcique α, le phosphate tricalcique β, le phosphate octocalcique et l'hydroxyapatite.

11. Acide pour utilisation selon l'une quelconque des revendications 1, 4 ou 5, sel pour utilisation selon l'une quelconque des revendications 1 à 5, ou composition pharmaceutique pour utilisation selon l'une quelconque des revendications 6 à 10, ledit acide, ledit sel ou ladite composition étant administré(e) localement dans une région d'un sujet.

12. Acide, sel ou composition pharmaceutique pour utilisation selon la revendication 10 ou 11, ladite région étant une région d'os alvéolaire.
